# FASCICULE DE BREVET EUROPEEN

(11) **EP 1 014 935 B1**
(45) Date de publication et mention de la délivrance du brevet: **04.12.2002**
(21) Numéro de dépôt: 98944002.9
(22) Date de dépôt: 16.09.1998
(51) Int. Cl.: A61K 7/48

(54) **UTILISATION DE L'ERGOSTEROL ET DES SES COMPOSES APPARENTES POUR STIMULER LA PROLIFERATION DES CELLULES DE LA PEAU**
VERWENDUNG VON ERGOSTEROL UND VON VERWANDTEN VERBINDUNGEN ZUR FÖRDERUNG DER PROLIFERATION VON HAUTZELLEN
USE OF ERGOSTEROL AND ITS APPARENT COMPOUNDS FOR STIMULATING THE PROLIFERATION OF SKIN CELLS

(30) Priorité: 18.09.1997 FR 9711655
(43) Date de publication de la demande: 05.07.2000
(73) Titulaire: LABORATOIRE DE BIOLOGIE VEGETALE YVES ROCHER, 56201 La Gacilly (FR)
(72) Inventeur: LUBRANO, Christian, F-94240 L'Haye-les-Roses (FR); POIRIER, Frédérique, F-75017 Paris (FR); ROBIN, Jean-Renaud, F-92000 Nanterre (FR)
(74) Mandataire: Bernasconi, Jean Raymond
(86) Numéro de dépôt international: FR9801983
(87) Numéro de publication internationale: WO99013858

(56) Documents cités:
- EP-A- 0 554 897
- FR-A- 1 594 884
- FR-A- 2 734 721
- US-A- 4 338 293
- PATENT ABSTRACTS OF JAPAN vol. 010, no. 112 (C-342), 25 avril 1986 & JP 60 239404 A (HITOSHI NAGAOKA), 28 novembre 1985
- PATENT ABSTRACTS OF JAPAN vol. 095, no. 007, 31 août 1995 & JP 07 101835 A (POLA CHEM IND INC), 18 avril 1995

## Description

La présente invention a trait à l'utilisation de composés de formule I, en particulier de l'ergostérol naturel ou obtenu par synthèse chimique, enzymatique, ou par voie biotechnologique, ou d'un extrait végétal, tel qu'un extrait de champignon, contenant de l'ergostérol, en particulier un extrait de *Lentinus edodes*, pour stimuler la prolifération des cellules épidermiques et des fibroblastes.

L'ergostérol, ou provitamine D2, est un composé à noyau stéroïde possédant des doubles liaisons Δ5 et Δ7 dans son cycle de base, présentant la formule suivante :

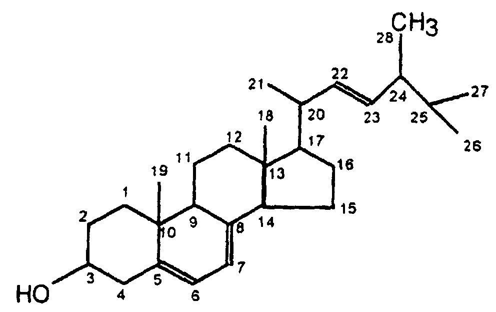

L'ergostérol pur est connu comme ingrédient dans des préparations cosmétiques.

Transformé en vitamine D sous l'action de la lumière, il est en particulier utilisé comme précurseur de cette vitamine dans des compositions cosmétiques dites "de jour" (FR 2 734 721).

En revanche l'action de l'ergostérol sur la prolifération des cellules épidermiques et des fibroblastes n'était jusqu'à maintenant pas connue.

L'ergostérol est naturellement présent dans le règne végétal, notamment chez les champignons. Il est ainsi présent dans les levures et est également abondant chez les champignons supérieurs tels que notamment *Lentinus edodes*, aussi appelé Shiitaké.

Il s'agit d'un champignon médicinal et alimentaire d'origine asiatique. Le nom japonais Shiitaké dérive de son association avec l'arbre "shiia", le chêne.

Ce champignon se trouve à l'état naturel sur des troncs d'arbres morts, notamment sur des chênes, frênes, marronniers, et est actuellement largement cultivé.

Les propriétés médicinales des champignons sont principalement reliées à l'un de ses polysaccharides, le lentinane grâce à une activité immunostimulante et à l'éritadénine qui présente une activité hypocholestérolémiante.

Le Shiitaké contient également des protéines (26 % par rapport à la matière sèche), des glucides (65 % de la matière sèche), des minéraux (calcium, potassium) et des vitamines, notamment les vitamines B2 et C.

D'autre part, une fraction lipidique du Shiitaké s'est révélée riche en ergostérol (Ying et al., 1987, Science Press).

Les auteurs de la présente invention se sont intéressés aux propriétés biologiques des dérivés de l'ergostérol, en particulier des composés stéroïdes possédant une double liaison Δ5 et une double liaison Δ7 répondant à la formule I suivante : 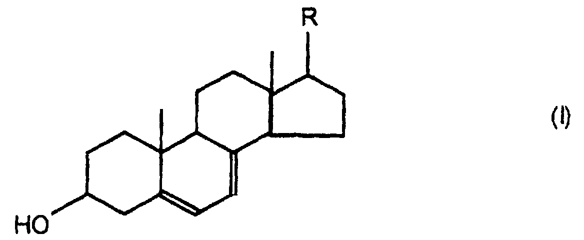 dans laquelle R représente une chaîne hydrocarbonée en C₁-C₁₂ linéaire ou ramifiée, substituée ou non substituée, saturée ou insaturée.

Par "chaîne substituée", on entend une chaîne hydrocarbonée substituée par un ou plusieurs groupements substituants, tels que notamment un groupement hydroxy ou un groupement alkoxy en C₁-C₈.

Par "chaîne insaturée", on entend une chaîne hydrocarbonée partiellement ou totalement insaturée.

Ils ont découvert que ces composés, et en particulier l'ergostérol d'origine synthétique ou sous forme d'extrait végétal, ont une action stimulante sur les cellules épidermiques et les fibroblastes, ce qui les rend particulièrement utiles dans des compositions cosmétiques destinées à lutter contre le vieillissement cutané et à améliorer la qualité structurelle de la peau.

La présente invention à donc pour objet l'utilisation cosmétique de composés de formule I telle que décrite précédemment, en tant que principes actifs, pour stimuler la prolifération des cellules épidermiques et des fibroblastes.

Parmi ces composés de formule I, l'ergostérol, d'origine synthétique ou naturelle, est préférentiellement utilisé.

Plus particulièrement, les auteurs de la présente invention ont mis en évidence qu'un extrait végétal, tel qu'un extrait lipidique de *Lentinus edodes*, avait même, en outre, une action sur la prolifération des cellules épidermiques et des fibroblastes supérieure à l'action de l'ergostérol pur d'origine synthétique.

La présente invention a donc pour objet l'utilisation cosmétique d'ergostérol sous la forme d'un extrait lipidique de *Lentinus edodes*, comme agent actif pour stimuler la prolifération des cellules épidermiques et des fibroblastes.

Un tel extrait végétal peut être obtenu par extraction par un solvant apolaire ou peu polaire ou un mélange apolaire ou peu polaire de solvants notamment choisis parmi le dichlorométhane, l'hexane, l'acétate d'éthyle, l'ethanol et l'acétone. Il peut être obtenu en particulier par extraction dans un mélange dichlorométhane/éthanol, mélange globalement peu polaire, dans des proportions d'environ 60/ environ 40 en volume. Cet extrait peut également être avantageusement obtenu à l'aide de dioxyde de carbone supercritique et est alors plus riche en ergostérol.

De manière préférentielle, les compositions selon l'invention peuvent comprendre de 0,0001 à 0,01% dudit extrait végétal exprimé en poids par rapport au poids de la composition.

Les compositions cosmétiques selon l'invention se présentent de préférence sous la forme d'une émulsion simple huile dans eau ou eau dans huile, ou d'une émulsion multiple, de micro-émulsion, de gel aqueux ou hydroalcoolique, de crème, d'huile, de lotion aqueuse ou hydroalcoolique, ou de bâtonnet.

La présente invention a également pour objet une méthode de traitement cosmétique consistant à appliquer sur la peau une composition cosmétique telle que décrite précédemment.

L'action des compositions selon l'invention intervient au niveau épidermique par augmentation de l'activité mitotique et du renouvellement des cellules épidermiques, aboutissant à un épaississement de l'épiderme. Au niveau dermique, les compositions selon l'invention agissent en stimulant les fibroblastes, ce qui accélère la maturation et le renouvellement cellulaire et qui se traduit par une synthèse accrue de collagène, une augmentation de la teneur en eau, une augmentation de l'épaisseur et une amélioration de l'élasticité de la peau.

Globalement, il apparaît qu'une action trophique intéressante peut être obtenue par application d'une composition cosmétique selon l'invention, et plus particulièrement un maintien ou une restauration du contenu collagénique de la peau.

Les exemples suivants illustrent la présente invention.

### EXEMPLE 1

### Préparation de l'extrait lipidique de Shiitaké

Le Shiitaké sec est réduit en poudre et mélangé au solvant (dichlorométhane/éthanol 60/40) dans une proportion de 10 % à 20 % par rapport au solvant et avantageusement à la teneur de 20 % (poids/volume).

La poudre est extraite sous reflux pendant deux heures.

L'extrait est ensuite filtré et le solvant est évaporé sous vide. Le résidu constitue l'extrait lipidique de Shiitaké.

L'extrait peut également être réalisé par extraction par un fluide supercritique, notamment le CO₂.

Cette extraction permet d'obtenir un extrait plus riche en ergostérol, de couleur moins foncée et d'odeur moins prononcée qu'un extrait réalisé à l'aide de solvants.

### EXEMPLE 2

### Dosage de l'ergostérol

Le dosage est réalisé par HPLC.

### a) Matériel/réactifs nécessaires

On utilise le matériel et produits suivants :
- Colonne MERCK Lichrocart Purospher RP 18 (5 microns) et précolonne
- acétonitrile, méthanol, chloroforme de qualité analytique
- témoin ergostérol SIGMA E6510.

### b) préparation de la phase mobile

On effectue le mélange acétonitrile/méthanol 60/40 (volume/volume, volumes mesurés séparément).

### c) préparation de l'échantillon

On reprend de l'extrait lipidique préparé dans l'exemple 1 dans un mélange chtoroforme/méthanol 50/50 (volume/volume). L'extrait est à concentrer ou à diluer selon les résultats obtenus.

On filtre par un filtre seringue de 0,45 µm.

### d) mode opératoire

Conditions HPLC :
- Température du four : 43°C
- Débit : 1 ml/min
- Longueur d'onde : 260 nm
- Pression indicative : 70 bars

### e) résultats

On réalise une droite étalon avec la substance témoin pour un dosage. Le temps de rétention indicatif est de onze minutes environ.

L'extrait lipidique obtenu par solvants présente une teneur de 5 % environ en ergostérol.

L'extrait obtenu par CO₂ supercritique présente une teneur de 9 % environ en ergostérol.

### EXEMPLE 3

### Tests sur cellules

### a) matériels et méthodes

L'efficacité de la phase lipophile de l'extrait de Shiitaké obtenu à l'aide de solvants a été testée sur des fibroblastes humains normaux (13^{ème} passage) en culture monocouche pour visualiser la prolifération des fibroblastes.

Les fibroblastes sont incubés pendant 24 heures à 37°C à 5 % de CO₂, dans des plaques à 24 puits, à une densité de 8000 cellules par cm². Le milieu de culture est composé de Dulbecco's Modified Eagle Medium sans rouge de phénol (DMEM, Gibco, référence 11880028), 10 % de sérum de veau nouveau-né (SVNN, Gibco, référence 16010084), 100 U/ml de pénicilline (Gibco, référence 15140106), 100 µg/ml de streptomycine (Gibco, référence 15140106), 2 mM de glutamine (Gibco, référence 250030024), 0,77 ml de glucose (Gibco, référence 19004019) pour 100 ml de milieu final et 1 % d'une solution d'acides aminés non essentiels (Gibco, référence 11140035).

L'extrait à tester est mis en contact avec les cellules dans le milieu de culture appauvri en SVNN (2 %). La concentration testée est de 0,015 µg/ml (1.5.10⁻⁶ %), l'extrait étant solubilisé dans l'éthanol préchauffé à 60°C.

Des contrôles positifs (milieu de culture à 10 % SVNN ; β-estradiol à 6.10⁻⁴µg/ml) et négatifs (milieu de culture à 2 % SVNN) sont réalisés en parallèle.

De la même manière on teste de l'ergostérol à 8.10⁻⁵µg/ml (SIGMA).

Les mesures sont faites à J1, J2 et J3 par un dosage au MTT (Sigma, M5655) qui permet de révéler l'activité des mitochondries. La solution de MTT est incubée pendant trois heures à 37°C. Le MTT est ensuite éliminé et du diméthylsulfoxide (DMSO, Sigma, D 5879) est déposé dans chaque puits pendant dix minutes. Après une agitation de quelques minutes, la lecture est faite à 570 nm.

### b) résultats

Après seulement trois jours de traitement, l'extrait testé présente une nette stimulation de la prolifération des fibroblastes. En effet, la phase lipophile du Shiitaké, à la concentration de 15.10⁻⁶ %, permet un gain de croissance de 27 % environ, après trois jours de traitement, pour les fibroblastes au 13^{ème} passage. En présence d'ergostérol pur, le gain de croissance est de 11 % à J3.

Parallèlement, en présence de β-estradiol, on obtient un gain de croissance des fibroblastes de 14 % à J3.

Ainsi, à une dose d'extrait lipophile de Shiitaké ou d'ergostérol pur comparable à celle du β-estradiol, on obtient une activité équivalente, voire supérieure sur la prolifération des fibroblastes humains.

### EXEMPLE 4

### Comparaison de l'action d'un extrait lipidique de Shiitaké obtenu par solvants (1), et par CO₂ supercritique (2)

### a) matériels et méthodes

La phase lipophile d'un extrait de Shiitaké obtenu par l'extraction au CO₂ supercritique a été testée. Le protocole de cette expérience est identique à celui décrit ci-dessus. Une mesure de la prolifération fibroblastique à J7 a en outre été réalisée. L'effet des extraits obtenus de manière différente (par solvants et par CO₂ supercritique) a été comparé.

Les deux extraits ont été testés aux concentrations de 0,015 µg/ml (1.5.10⁻⁶ %) et 0,0015 µg/ml (1.5.10⁻⁷ %), dans des conditions strictement identiques aux précédentes. Parallèlement, le β-oestradiol et l'ergostérol ont aussi été testés, toujours dans les mêmes conditions.

### b) résultats

Les meilleurs résultats sont obtenus après 7 jours de traitement avec les extraits utilisés à la concentration de 0,0015 µg/ml (1,5.10⁻⁷ %), pour les deux lots de fibroblastes. Cependant, il apparaît très nettement que ce sont les fibroblastes les plus âgés (12^{ème} passage) qui répondent le mieux aux extraits et plus particulièrement à la phase lipophile de Shiitaké obtenue par l'extraction au CO₂ supercritique. En effet, à ce stade, on peut observer un gain de croissance de 18,5 % alors que ce gain de croissance n'est que de 4 % pour les fibroblastes plus jeunes (6^{ème} passage). Aussi, il est à noter que le β-oestradiol et l'ergostérol ne fournissent respectivement des gains de croissance que de 13 % et 8 % à J7 pour les fibroblastes les plus âgés ; pour les fibroblastes les plus jeunes, nous obtenons 7 % de gain de croissance pour le β-oestradiol et 2 % pour l'ergostérol, à J7 toujours (cf tableau).

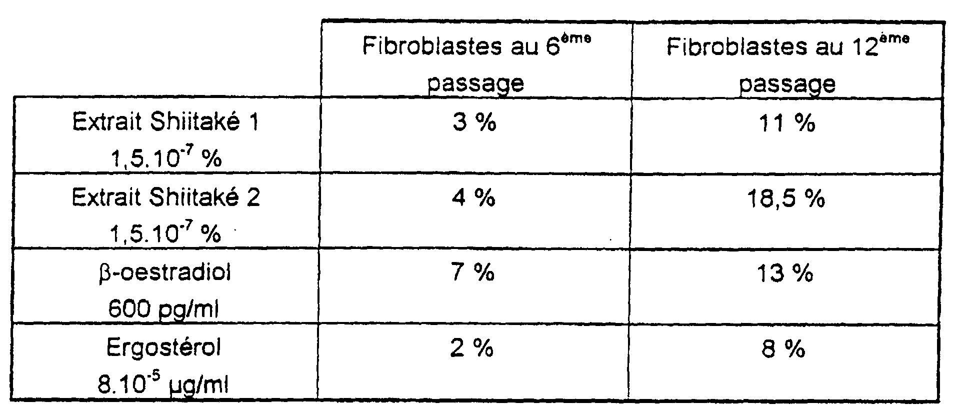

Ces résultats sont obtenus après 7 jours de traitement des fibroblastes par une phase lipophile de Shiitaké obtenue par solvant (n° 1) et une phase lipophile de Shiitaké obtenue par extraction au CO₂ supercritique (n° 2).

### EXEMPLE 5

### Formules incluant l'extrait lipidique de Shiitaké

### a) exemple de composition sous forme d'émulsion

| | |
|---|---|
| Eau | QSP |
| extrait lipidique de Shiitaké | 0,0001 % à 0,01 % |
| conservateurs | QSP |
| Propylèneglycol | 5,00 % |
| Gomme xanthane | 0,30 % |
| Copolymère acrylique/acrylate | 0,50 % |
| acide stéarique 100 OE** | 3,00 % |
| Stéarate de sorbitan | 2,00 % |
| Sorbitan laurate 20 OE | 3,00 % |
| alcool cétylstéarique | 1,50 % |
| Cire d'abeille | 1,00 % |
| Huile de germe de blé | 5,00 % |
| Diméthicone | 2,00 % |
| Cyclométhicone | 5,00 % |
| Gel de polyacrylamide | 2,00 % |
| Parfum | |

| | |
|---|---|
| ** acide stéarique avec 100 moles d'OE | |

### b) exemple de composition sous forme de crème

| | |
|---|---|
| Eau | QSP |
| Gomme xanthane | 0,30 % |
| Séquestrant (par ex. EDTA) | 0,05 % |
| Conservateurs | QSP |
| Extrait lipidique de Shiitaké | 0,0001 % à 0,01 % |
| Acide C 18 | 2,50 % |
| Alcool C 16 | 2,50 % |
| Trilaurine | 1,00 % |
| Beurre de karité | 3,00 % |
| Acétate de tocophérol | 0,05 % |
| β-bisabolol | 0,05 % |
| Huile végétale (blé) | 5,00 % |
| Diméthicone | 3,00 % |
| Acide polyacrylique | 0,30 % |
| Eau | 3,00 % |
| TEA (Triéthanolamine) | 1,50 % |
| Parfum | 0,10% |

### c) exemple de composition sous forme de lotion

| | |
|---|---|
| Eau | QSP |
| Agent séquestrant | 0,05 % |
| Propylèneglycol | 2,00 % |
| Conservateurs | QSP |
| Extrait lipidique de Shiitaké | 0,0001 % à 0,01 % |
| Alcool | 5 à 50 % |
| Alcool oléique 20 OE | 1,00 % |
| Parfum | 0,05 % |
| colorants | QSP |

## Revendications

1. Utilisation cosmétique de composés de formule I : 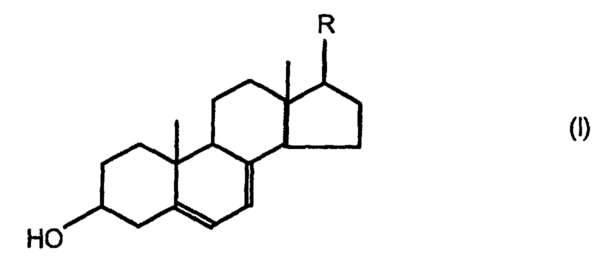 dans laquelle R représente une chaîne latérale hydrocarbonée en C₁-C₁₂ linéaire ou ramifiée, substituée ou non substituée, saturée ou insaturée, comme agent actif pour stimuler la prolifération des cellules épidermiques et des fibroblastes.

2. Utilisation selon la revendication 1, dans laquelle le composé de formule I est l'ergostérol d'origine synthétique ou naturelle.

3. Utilisation selon la revendication 2 dans laquelle l'ergostérol est présent sous la forme d'un extrait végétal, tel qu'un extrait de champignon, contenant de l'ergostérol.

4. Utilisation selon la revendication 3, dans laquelle ledit extrait est un extrait lipidique de *Lentinus edodes.*

5. Utilisation selon l'une quelconque des revendications 3 ou 4, dans laquelle l'extrait végétal est obtenu par extraction par un solvant ou un mélange de solvants choisis parmi le dichlorométhane, l'hexane, l'acétate d'éthyle, l'éthanol et l'acétone.

6. Utilisation selon la revendication 5, dans laquelle l'extrait végétal est obtenu par extraction dans un mélange dichlorométhane/éthanol dans des proportions d'environ 60/ environ 40 en volume.

7. Utilisation selon l'une quelconque des revendications 4 à 6, dans laquelle ledit extrait végétal est obtenu par mélange de 10 à 20% en poids de *Lentinus edodes* séché dans un solvant.

8. Utilisation selon l'une quelconque des revendications 3 ou 4, dans laquelle l'extrait végétal est obtenu par extraction à l'aide de dioxyde de carbone supercritique.

9. Utilisation selon l'une quelconque des revendications 3 à 8, dans laquelle l'ergostérol est sous la forme d'une composition comprenant de 0,0001 à 0,01% en poids dudit extrait végétal par rapport au poids de la composition.

10. Utilisation selon l'une des revendications 1 à 9, pour lutter contre le vieillissement cutané.

## Patentansprüche

1. Kosmetische Verwendung von Verbindungen mit der Formel I 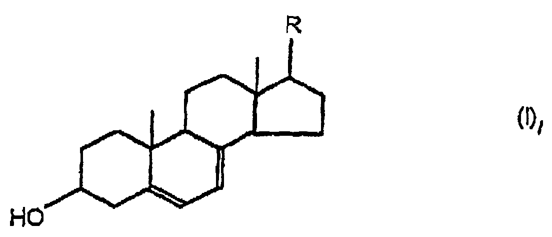 in welcher R eine geradkettige oder verzweigte C₁- bis C₁₂-Kohlenwasserstoffseitenkette bedeutet, die gegebenenfalls substituiert und gegebenenfalls ungesättigt ist, als Wirkstoff zur Stimulation der Vermehrung der Epidermiszellen und der Fibroblasten.

2. Verwendung nach Anspruch 1, bei welcher die Verbindung mit der Formel I Ergosterin synthetischer oder natürlicher Herkunft ist.

3. Verwendung nach Anspruch 2, bei welcher das Ergosterin in Form eines Ergosterin enthaltenden Pflanzenextrakts wie eines Pilzextrakts vorliegt.

4. Verwendung nach Anspruch 3, wobei der Extrakt ein Lipidextrakt von *Lentinus edodes* ist.

5. Verwendung nach Anspruch 3 oder 4, wobei der Pflanzenextrakt durch Extraktion mit einem Lösungsmittel oder Gemisch von Lösungsmitteln, das/die aus Dichlormethan, Hexan, Ethylacetat, Ethanol und Aceton ausgewählt ist/sind, erhalten worden ist.

6. Verwendung nach Anspruch 5, wobei der Pflanzenextrakt durch Extraktion mit einem Dichlormethan/Ethanol-Gemisch mit einem Volumenverhältnis von etwa 60/etwa 40 erhalten worden ist.

7. Verwendung nach einem der Ansprüche 4 bis 6, bei welcher der Pflanzenextrakt durch Mischen von 10 bis 20 Gew.% getrocknetem *Lentinus edodes* in ein Lösungsmittel erhalten worden ist.

8. , Verwendung nach Anspruch 3 oder 4, bei welcher der Pflanzenextrakt durch Extraktion mit überkritischem Kohlendioxid erhalten worden ist.

9. Verwendung nach einem der Ansprüche 3 bis 8, bei welcher das Brgosterin in Form einer Zusammensetzung vorliegt, die 0,0001 bis 0,01 Gew.% Pflanzenextrakt, bezogen auf das Gewicht der Zusammensetzung, enthält.

10. Verwendung nach einem der Ansprüche 1 bis 9 zur Bekämpfung der Hautalterung.

## Claims

1. Cosmetic use of compounds of formula I: 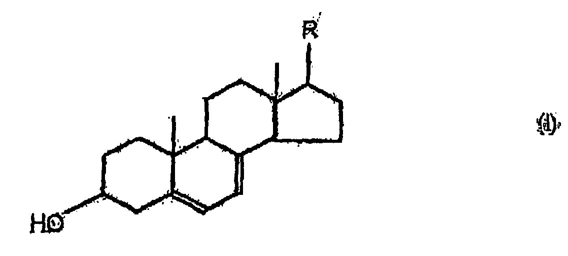 in which R represents a straight or branched, substituted or unsubstituted, saturated or unsaturated side C₁-C₁₂ hydrocarbon chain as an active agent for stimulating the proliferation of epidermic cells and fibroblasts.

2. Use according to claim 1 wherein the compound of formula I is ergosterol of synthetic or natural origin.

3. Use according to claim 2 wherein the ergosterol is present in the form of a plant extract such as a mushroom extract containing ergosterol.

4. Use according to claim 3 wherein said extract is a lipidic extract of *Lentinus edodes*.

5. Use according to either one of claims 3 and 4 wherein the plant extract is obtained by extraction by a solvent or a mixture of solvents selected from dichloromethane, hexane, ethyl acetate, ethanol or acetone.

6. Use according to claim 5 wherein the plant extract is obtained by extraction in a dlchloromethane/ethanol mixture in proportions of about 60/about 40 by volume.

7. Use according to any one of claims 4 to 6 wherein said plant extract is obtained by mixing from 10 to 20% by weight of dried *Lentinus edodes* in a solvent.

8. Use according to either one of claims 3 and 4 wherein the plant extract is obtained by extraction by means of supercritical carbon dioxide.

9. Use according to any one of claims 3 to 8 wherein the ergosterol is in the form of a composition comprising from 0.0001 to 0.01% by weight of said plant extract with respect to the weight of the composition.

10. Use according to any one of claims 1 to 9 for combating skin ageing.
